# EUROPEAN PATENT SPECIFICATION

(11) **EP 1 993 590 B1**
(45) Date of publication and mention of the grant of the patent: **25.12.2013**
(21) Application number: 07752090.6
(22) Date of filing: 27.02.2007
(51) Int. Cl.: A01N 43/04, A61K 38/18, A61P 25/02

(54) **COMPOSTIONS AND METHODS FOR ADMINISTERING GDNF LIGAND FAMILY PROTEINS**
ZUSAMMENSETZUNGEN UND VERFAHREN ZUR VERABREICHUNG VON PROTEINEN DER GDNF-LIGANDEN-FAMILIE
COMPOSITIONS ET PROCEDES D'ADMINISTRATION DE PROTEINES DE LA FAMILLE DES LIGANDS GDNF

(30) Priority: 01.03.2006 US 778509 P
(43) Date of publication of application: 26.11.2008
(73) Proprietor: Biogen Idec MA Inc., Cambridge, Massachusetts 02142 (US)
(72) Inventor: ROSSOMANDO, Anthony, South Grafton, MA 01560 (US); PEPINSKY, R., Blake, Arlington, MA 02474 (US)
(74) Representative: Pohlman, Sandra M.
(86) International application number: PCT/US2007/005366
(87) International publication number: WO 2007/103182

(56) References cited:
- WO-A-01/66164
- WO-A-02/060929
- US-A1- 2002 114 780
- US-A1- 2002 114 780

## Description

### Technical Field

The invention relates to protein chemistry, molecular biology, and neurobiology.

### Background

Glial cell line-derived neurotrophic factor (GDNF) was initially identified as a critical factor for the survivability of dopaminergic neurons in the midbrain. The pattern of the seven cysteine residues within its amino acid sequence was consistent with that of transforming growth factor-beta (TGF-beta), indicating that GDNF (and related proteins) may be considered a subclass of this "superfamily" (Lin et al., 1993, Science, 260:1130). The characterization of GDNF was followed by the identification of the related growth factors Neurturin (Kotzbauer et al., 1996, Nature, 384:467), Persephin (Milbrandt et al., Neuron, 20:245), and Neublastin (also known as Artemin and Enovin) (Baloh et al., 1998, Neuron, 21:1291; Masure et al., 1999, Eur J Biochem, 266:892), which together comprise the GDNF ligand family of neurotrophic factors.

Studies with GDNF knock-out mice suggested a major role of GDNF is in the development of the enteric nervous system and regulation of renal organogenesis (Moore et al., 1996, Nature, 382:76). Neurturin was first characterized in studies aimed at examination of recombinant growth factors expressed in transfected Chinese hamster ovary (CHO) cells when it was found that one of these factors enhanced the survival of sympathetic neurons cultured from neonatal mouse superior cervical ganglia even in the presence of antiserum to NGF (Kotzbauer et al., *supra).* Expression cloning studies led to the characterization of Persephin which, like GDNF, promoted survival of cultured motor neurons and of midbrain dopaminergic neurons and promoted regeneration of axotomized motor neurons in neonatal rats (Milbrandt et al., *supra).* The most recently discovered GDNF ligand family member is Neublastin, which promotes the outgrowth and survival of neurons of the peripheral and central nervous system (Baudet et al., 2000, Development, 127:4335; Masure et al., *supra;* Rosenblad et al., 2000, Mol. Cell Neurosci., 15(2):199).

All of the GDNF ligand family members act through ternary complex receptor systems containing the RET receptor tyrosine kinase as common signaling component (Baloh et al., 1998, Neuron, 21:1291; Mason, et al., 2000, Pharm Acta Helv, 74:261; Masure et al., 2000, J Biol Chem, 275:39427). Specificity is conferred by binding of the ligands to a unique GDNF family receptor alpha (GFR alpha). The GFR alpha 1 to GFR alpha 4 receptors are glycosyl-phosphatidyl inositol (GPI) anchored proteins that, when bound to the preferred GDNF ligand, activate RET. GDNF binds preferentially to GFR alpha 1, Neurturin to GFR alpha 2, Neublastin to GFR alpha 3, and Persephin to GFR alpha 4.

### Summary

The invention is based, at least in part, on the discovery that co-administration of heparin with a systemically delivered GDNF ligand family protein (Neublastin) increases serum exposure of the administered protein.

Disclosed are methods of increasing serum exposure of an administered GDNF ligand family protein by administering to a subject via systemic delivery a pharmaceutical composition containing (i) a GDNF ligand family protein, and (ii) an amount of heparin or heparan sulphate that increases serum exposure of the administered GDNF ligand family protein in the subject.

As used herein, a "GDNF ligand family protein" refers to a Neublastin polypeptide, a GDNF polypeptide, a Neurturin polypeptide, or a Persephin polypeptide.

As used herein, "an amount of heparin or heparan sulphate that increases serum exposure of the administered GDNF ligand family protein" refers to an amount that results in serum levels of the protein following administration that exceed serum levels that result when the GDNF ligand family protein is administered, via the same route of administration, in the absence of heparin or heparan sulphate.

"Systemic delivery" refers to a route of administration that results in the administered protein traveling through the bloodstream and reaching cells throughout the body. Systemic delivery does not encompass localized means of delivery such as intracerebral delivery, intraventricular delivery, or intracerebroventricular delivery.

The invention relates to uses for treating a nervous system disorder by administering to a subject that has a nervous system disorder, via systemic delivery, an effective amount of a pharmaceutical composition containing (i) a GDNF ligand family protein, and (ii) an amount of heparin or heparan sulphate that increases serum exposure of the administered GDNF ligand family protein in the subject. The nervous system disorder can be, for example, neuropathic pain or loss of pain sensitivity associated with a neuropathy. Additional examples of nervous system disorders that can be treated according to the methods are detailed herein.

In some embodiments of the uses described herein, the systemic delivery is intravenous administration. In some embodiments, the systemic delivery is subcutaneous administration.

In some embodiments of the uses described herein, the GDNF ligand family protein is a Neublastin polypeptide. The Neublastin polypeptide can, for example, contain an amino acid sequence that is at least 80% identical to amino acids 15-113 of SEQ ID NO:1, wherein the polypeptide, when dimerized, binds to a complex containing GFRalpha3 and RET. In some embodiments, the amino acid sequence is at least 90%, 95%, or 98% identical to amino acids 15-113 of SEQ ID NO:1. In some embodiments, the amino acid sequence is at least 90%, 95%, or 98% identical to SEQ ID NO:1. In some embodiments, the Neublastin polypeptide contains or consists of amino acids 15-113 of SEQ ID NO:1, amino acids 10-113 of SEQ ID NO:1, or the amino acid sequence of SEQ ID NO:1.

In some embodiments of the uses described herein, the GDNF ligand family protein is a GDNF polypeptide. The GDNF polypeptide can, for example, contain an amino acid sequence that is at least 80% identical to SEQ ID NO:2, wherein the polypeptide, when dimerized, binds to a complex containing GFRalpha1 and RET. In some embodiments, the amino acid sequence is at least 90%, 95%, or 98% identical to SEQ ID NO:2. In some embodiments, the GDNF polypeptide contains or consists of the amino acid sequence of SEQ ID NO:2.

In some embodiments of the uses described herein, the GDNF ligand family protein is a Neurturin polypeptide. The Neurturin polypeptide can, for example, contain an amino acid sequence that is at least 80% identical to SEQ ID NO:3, wherein the polypeptide, when dimerized, binds to a complex containing GFRalpha2 and RET. In some embodiments, the amino acid sequence is at least 90%, 95%, or 98% identical to SEQ ID NO:3. In some embodiments, the Neurturin polypeptide contains or consists of the amino acid sequence of SEQ ID NO:3.

In some embodiments of the uses described herein, the GDNF ligand family protein is a Persephin polypeptide. The Persephin polypeptide can, for example, contain an amino acid sequence that is at least 80% identical to SEQ ID NO:4, wherein the polypeptide, when dimerized, binds to a complex containing GFRalpha4 and RET. In some embodiments, the amino acid sequence is at least 90%, 95%, or 98% identical to SEQ ID NO:4. In some embodiments, the Persephin polypeptide contains or consists of the amino acid sequence of SEQ ID NO:4.

In some embodiments of the uses described herein, the GDNF ligand family protein is not conjugated to a polymer (e.g., a polyalkylene glycol such as polyethylene glycol). For example, the Neublastin polypeptide can be a non-polymer-conjugated Neublastin polypeptide.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, the exemplary methods and materials are described below. In case of conflict, the present application, including definitions, will control.

Other features and advantages of the invention will be apparent from the following detailed description, and from the claims.

### Brief Description of the Drawings

Fig. 1 is an alignment of wild type human (SEQ ID NO:5), mouse (SEQ ID NO:6), and rat (SEQ ID NO:7) pre pro Neublastin polypeptides. The left and right vertical lines indicate, respectively, the start of the 113 amino acid and 104 amino acid forms. The RRXR heparin binding motif is boxed.
Fig. 2 is an alignment of wild type human (SEQ ID NO:8), mouse (SEQ ID NO:9), and rat (SEQ ID NO:10) pre pro GDNF polypeptides. The amino acid residue at the start of the mature form of the protein is bolded and underlined.
Fig. 3 is an alignment of wild type human (SEQ ID NO:11), mouse (SEQ ID NO:12), and rat (SEQ ID NO:13) pre pro Neurturin polypeptides. The amino acid residue at the start of the mature form of the protein is bolded and underlined.
Fig. 4 is an alignment of wild type human (SEQ ID NO:14), mouse (SEQ ID NO:15), and rat (SEQ ID NO:16) pre pro Persephin polypeptides. The amino acid residue at the start of the mature form of the protein is bolded and underlined.

### Detailed Description

The GDNF ligand family of proteins contains the following four members: Neublastin, GDNF, Neurturin, and Persephin. The present invention provides methods for increasing serum exposure of a systemically delivered GDNF ligand family protein (or a biologically active variant thereof) by co-administration of heparin or heparan sulphate with the protein. As disclosed in the accompanying example, co-administration of heparin with Neublastin was found to increase the area under the curve and enhance the half life of the systemically administered protein.

### Neublastin Polypeptides

Mature wild type human Neublastin is 113 amino acids in length and has the following amino acid sequence: AGGPGSRARAAGARGCRLRSQLVPVRALGLG HRSDELVRFRFCSGSCRRARSPHDLSLASLLGAGALRPPPGSRPVSQPCCRPTR YEAVSFMDVNSTWRTVDRLSATACGCLG (SEQ ID NO:1). Polypeptides having the amino acid sequence of SEQ ID NO:1 or biologically active variants thereof can be used in the methods described herein. A variant Neublastin polypeptide can contain one or more additions, substitutions, and/or deletions, as detailed in the following sections. Wild-type Neublastin polypeptides and biologically active variants thereof are collectively referred to herein as "Neublastin polypeptides."

A variant Neublastin polypeptide can vary in length from the corresponding wild-type polypeptide. Although the mature human Neublastin polypeptide (SEQ ID NO:1) consists of the carboxy terminal 113 amino acids of pre pro Neublastin (SEQ ID NO:5), not all of the 113 amino acids are required to achieve useful Neublastin biological activity. Amino terminal truncation is permissible. Thus, a variant Neublastin polypeptide can contain, for example, the carboxy terminal 99, 100, 101, 102, 103, 104, 105, 106, 107, 108, 109, 110, 111, 112, or 113 amino acids of SEQ ID NO:1 (i.e., its length can be 99, 100, 101, 102, 103, 104, 105, 106, 107, 108, 109, 110, 111, 112, or 113 amino acids).

A variant Neublastin polypeptide can also vary in sequence from the corresponding wild-type polypeptide. In particular, certain amino acid substitutions can be introduced into the Neublastin sequence without appreciable loss of a Neublastin biological activity. In exemplary embodiments, a variant Neublastin polypeptide (i) contains one or more amino acid substitutions, and (ii) is at least 70%, 80%, 85%, 90%, 95%, 98% or 99% identical to SEQ ID NO:1 (or 70%, 80%, 85%, 90%, 95%, 98% or 99% identical to amino acids 15-113 of SEQ ID NO:1). A variant Neublastin polypeptide differing in sequence from SEQ ID NO:1 (or differing in sequence from amino acids 15-113 of SEQ ID NO:1) may include one or more amino acid substitutions (conservative or non-conservative), one or more deletions, and/or one or more insertions.

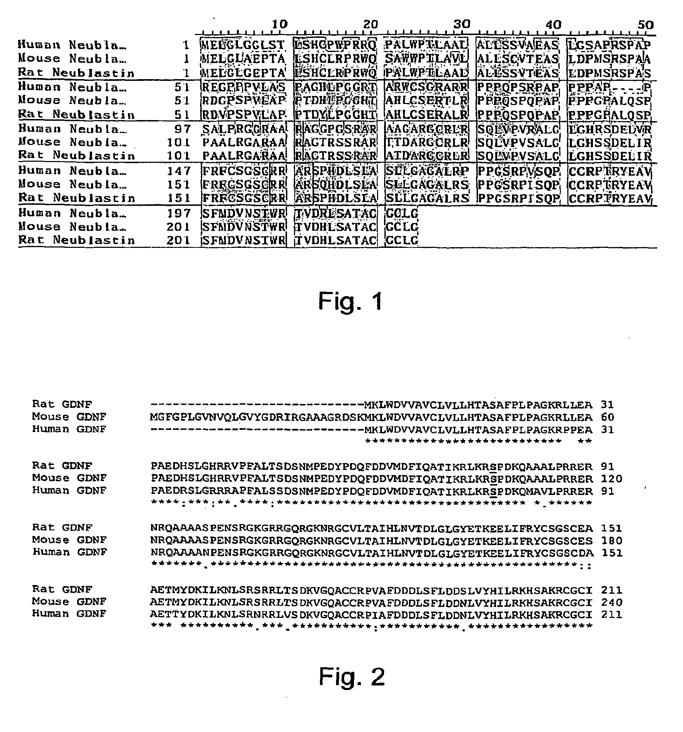

Fig. 1 is an alignment of the wild type human, mouse, and rat pre pro Neublastin polypeptides. The vertical lines in Fig. 1 indicate the start of the mature 113 amino acid form (left vertical line) and 104 amino acid form (right vertical line) of Neublastin. The RRXR heparin binding motif is boxed. This alignment of naturally occurring, bioactive forms of Neublastin indicates specific exemplary residues (i.e., those that are not conserved among the human, mouse, and rat forms) that can be substituted without eliminating bioactivity.

Percent identity between amino acid sequences can be determined using the BLAST 2.0 program. Sequence comparison can be performed using an ungapped alignment and using the default parameters (Blossom 62 matrix, gap existence cost of 11, per residue gap cost of 1, and a lambda ratio of 0.85). The mathematical algorithm used in BLAST programs is described in Altschul et al., 1997, Nucleic Acids Research 25:3389-3402.

A conservative substitution is the substitution of one amino acid for another with similar characteristics. Conservative substitutions include substitutions within the following groups: valine, alanine and glycine; leucine, valine, and isoleucine; aspartic acid and glutamic acid; asparagine and glutamine; serine, cysteine, and threonine; lysine and arginine; and phenylalanine and tyrosine. The non-polar hydrophobic amino acids include alanine, leucine, isoleucine, valine, proline, phenylalanine, tryptophan and methionine. The polar neutral amino acids include glycine, serine, threonine, cysteine, tyrosine, asparagine and glutamine. The positively charged (basic) amino acids include arginine, lysine and histidine. The negatively charged (acidic) amino acids include aspartic acid and glutamic acid. Any substitution of one member of the above-mentioned polar, basic or acidic groups by another member of the same group can be deemed a conservative substitution.

Non-conservative substitutions include those in which (i) a residue having an electropositive side chain (e.g., Arg, His or Lys) is substituted for, or by, an electronegative residue (e.g., Glu or Asp), (ii) a hydrophilic residue (e.g., Ser or Thr) is substituted for, or by, a hydrophobic residue (e.g., Ala, Leu, Ile, Phe or Val), (iii) a cysteine or proline is substituted for, or by, any other residue, or (iv) a residue having a bulky hydrophobic or aromatic side chain (e.g., Val, Ile, Phe or Trp) is substituted for, or by, one having a smaller side chain (e.g., Ala, Ser) or no side chain (e.g., Gly).

A biologically active variant Neublastin polypeptide, when dimerized, binds to a ternary complex containing GFRalpha3 and RET. Any method for detecting binding to this complex can be used to evaluate the biological activity a variant Neublastin polypeptide. Exemplary assays for detecting the ternary complex-binding ability of a variant Neublastin polypeptide are described in WO00/01815.

A variant Neublastin polypeptide can also be assessed to evaluate its ability to trigger the Neublastin signaling cascade. For example, the Kinase Receptor Activation (KIRA) assay can be used to assess the ability of a variant Neublastin polypeptide to induce RET autophosphorylation *(See also,* Sadick et al., 1996, Anal. Biochem., 235(2):207).

### GDNF Polypeptides

Mature wild type human GDNF is 134 amino acids in length and has the following amino acid sequence: SPDKQMAVLPRRERNRQAAAANPENSRGKGR RGQRGKNRGCVLTAIHLNVTDLGLGYETKEELIFRYCSGSCDAAETTYDKILK NLSRNRRLVSDKVGQACCRPIAFDDDLSFLDDNLVYHILRKHSAKRCGCI (SEQ ID NO:2). Polypeptides having the amino acid sequence of SEQ ID NO:2 or biologically active variants thereof can be used in the methods described herein. A variant GDNF polypeptide can contain one or more additions, substitutions, and/or deletions, as detailed in the following sections. Wild-type GDNF polypeptides and biologically active variants thereof are collectively referred to herein as "GDNF polypeptides."

A variant GDNF polypeptide can vary in length from the corresponding wild-type polypeptide. Although the mature human GDNF polypeptide (SEQ ID NO:2) consists of the carboxy terminal 134 amino acids of pre pro GDNF (SEQ ID NO:8), not all of the 134 amino acids are required to achieve useful GDNF biological activity (e.g., amino terminal truncation is permissible).

A variant GDNF polypeptide can also vary in sequence from the corresponding wild-type polypeptide. In particular, certain amino acid substitutions can be introduced into the GDNF sequence without appreciable loss of a GDNF biological activity. In exemplary embodiments, a variant GDNF polypeptide (i) contains one or more amino acid substitutions, and (ii) is at least 70%, 80%, 85%, 90%, 95%, 98% or 99% identical to SEQ ID NO:2. A variant GDNF polypeptide differing in sequence from SEQ ID NO:2 may include one or more amino acid substitutions (conservative or non-conservative), one or more deletions, and/or one or more insertions.

Fig. 2 is an alignment of the wild type human, mouse, and rat pre pro GDNF polypeptides. The amino acid residue underlined and bolded in Fig. 2 indicates the start of the mature 134 amino acid form of GDNF. This alignment of naturally occurring, bioactive forms of GDNF indicates specific exemplary residues (i.e., those that are not conserved among the human, mouse, and rat forms) that can be substituted without eliminating bioactivity.

A biologically active variant GDNF polypeptide, when dimerized, binds to a ternary complex containing GFRalpha1 and RET. Any method for detecting binding to this complex can be used to evaluate the biological activity a variant GDNF polypeptide. Exemplary assays for detecting the ternary complex-binding ability of a variant GDNF polypeptide are described in WO00/01815.

A variant GDNF polypeptide can also be assessed to evaluate its ability to trigger the GDNF signaling cascade. For example, the KIRA assay can be used to assess the ability of a variant GDNF polypeptide to induce RET autophosphorylation *(See also,* Sadick et al., 1996, Anal. Biochem., 235(2):207).

### Neurturin Polypeptides

Mature wild type human Neurturin is 102 amino acids in length and has the following amino acid sequence: ARLGARPCGLRELEVRVSELGLGYASDETVLF RYCAGACEAAARVYDLGLRRLRQRRRLRRERVRAQPCCRPTAYEDEVSFLD AHSRYHTVHELSARECACV (SEQ ID NO:3). Polypeptides having the amino acid sequence of SEQ ID NO:3 or biologically active variants thereof can be used in the methods described herein. A variant Neurturin polypeptide can contain one or more additions, substitutions, and/or deletions, as detailed in the following sections. Wild-type Neurturin polypeptides and biologically active variants thereof are collectively referred to herein as "Neurturin polypeptides."

A variant Neurturin polypeptide can vary in length from the corresponding wild-type polypeptide. Although the mature human Neurturin polypeptide (SEQ ID NO:3) consists of the carboxy terminal 102 amino acids of pre pro Neurturin (SEQ ID NO:11), not all of the 102 amino acids are required to achieve useful Neurturin biological activity (e.g., amino terminal truncation is permissible).

A variant Neurturin polypeptide can also vary in sequence from the corresponding wild-type polypeptide. In particular, certain amino acid substitutions can be introduced into the Neurturin sequence without appreciable loss of a Neurturin biological activity. In exemplary embodiments, a variant Neurturin polypeptide (i) contains one or more amino acid substitutions, and (ii) is at least 70%, 80%, 85%, 90%, 95%, 98% or 99% identical to SEQ ID NO:3. A variant Neurturin polypeptide differing in sequence from SEQ ID NO:3 may include one or more amino acid substitutions (conservative or non-conservative), one or more deletions, and/or one or more insertions.

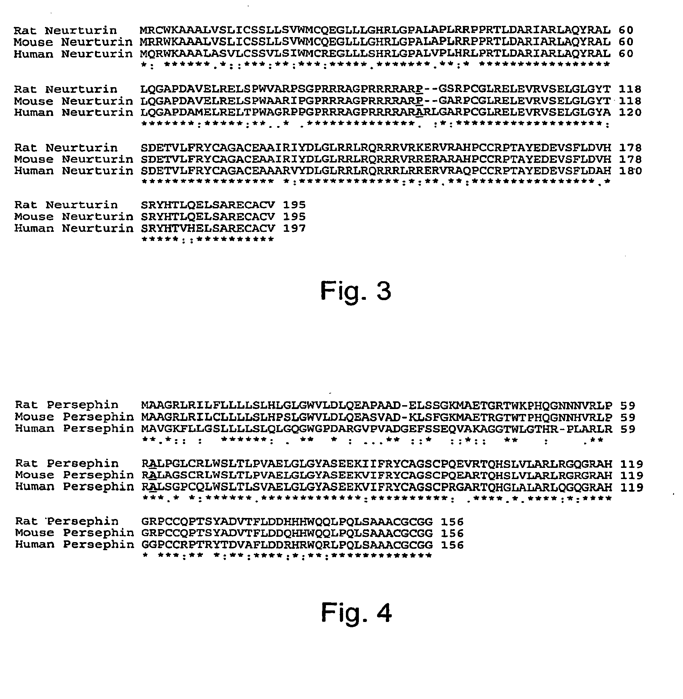

Fig. 3 is an alignment of the wild type human, mouse, and rat pre pro Neurturin polypeptides. The amino acid residue underlined and bolded in Fig. 3 indicates the start of the mature 102 amino acid form of Neurturin. This alignment of naturally occurring, bioactive forms of Neurturin indicates specific exemplary residues (i.e., those that are not conserved among the human, mouse, and rat forms) that can be substituted without eliminating bioactivity.

A biologically active variant Neurturin polypeptide, when dimerized, binds to a ternary complex containing GFRalpha2 and RET. Any method for detecting binding to this complex can be used to evaluate the biological activity a variant Neurturin polypeptide. Exemplary assays for detecting the ternary complex-binding ability of a variant Neurturin polypeptide are described in WO00/01815.

A variant Neurturin polypeptide can also be assessed to evaluate its ability to trigger the Neurturin signaling cascade. For example, the KIRA assay can be used to assess the ability of a variant Neurturin polypeptide to induce RET autophosphorylation *(See also,* Sadick et al., 1996, Anal. Biochem., 235(2):207).

### Persephin Polypeptides

Mature wild type human Persephin is 96 amino acids in length and has the following amino acid sequence: ALSGPCQLWSLTLSVAELGLGYASEEKVIFRY CAGSCPRGARTQHGLALARLQGQGRAHGGPCCRPTRYTDVAFLDDRHRWQ RLPQLSAAACGCGG (SEQ ID NO:4). Polypeptides having the amino acid sequence of SEQ ID NO:4 or biologically active variants thereof can be used in the methods described herein. A variant Persephin polypeptide can contain one or more additions, substitutions, and/or deletions, as detailed in the following sections. Wild-type Persephin polypeptides and biologically active variants thereof are collectively referred to herein as "Persephin polypeptides."

A variant Persephin polypeptide can vary in length from the corresponding wild-type polypeptide. Although the mature human Persephin polypeptide (SEQ NO:4) consists of the carboxy terminal 96 amino acids of pre pro Persephin (SEQ ID NO:14), not all of the 96 amino acids are required to achieve useful Persephin biological activity (e.g., amino terminal truncation is permissible).

A variant Persephin polypeptide can also vary in sequence from the corresponding wild-type polypeptide. In particular, certain amino acid substitutions can be introduced into the Persephin sequence without appreciable loss of a Persephin biological activity. In exemplary embodiments, a variant Persephin polypeptide (i) contains one or more amino acid substitutions, and (ii) is at least 70%, 80%, 85%, 90%, 95%, 98% or 99% identical to SEQ ID NO:4. A variant Persephin polypeptide differing in sequence from SEQ ID NO:4 may include one or more amino acid substitutions (conservative or non-conservative), one or more deletions, and/or one or more insertions.

Fig. 4 is an alignment of the wild type human, mouse, and rat pre pro Persephin polypeptides. The amino acid residue underlined and bolded in Fig. 4 indicates the start of the mature 96 amino acid form of Persephin. This alignment of naturally occurring, bioactive forms of Persephin indicates specific exemplary residues (i.e., those that are not conserved among the human, mouse, and rat forms) that can be substituted without eliminating bioactivity.

A biologically active variant Persephin polypeptide, when dimerized, binds to a ternary complex containing GFRalpha4 and RET. Any method for detecting binding to this complex can be used to evaluate the biological activity a variant Persephin polypeptide. Exemplary assays for detecting the ternary complex-binding ability of a variant Persephin polypeptide are described in WO00/01815.

A variant Persephin polypeptide can also be assessed to evaluate its ability to trigger the Persephin signaling cascade. For example, the KIRA assay can be used to assess the ability of a variant Persephin polypeptide to induce RET autophosphorylation *(See also,* Sadick et al., 1996, Anal. Biochem., 235(2):207).

### Preparation of GDNF Ligand Family Proteins

A GDNF ligand family protein (e.g., a Neublastin polypeptide, a GDNF polypeptide, a Neurturin polypeptide, or a Persephin polypeptide described herein) can optionally contain heterologous amino acid sequences in addition to a GDNF ligand family protein. "Heterologous," as used when referring to an amino acid sequence, refers to a sequence that originates from a source foreign to the particular host cell, or, if from the same host cell, is modified from its original form. Exemplary heterologous sequences include a heterologous signal sequence (e.g., native rat albumin signal sequence, a modified rat signal sequence, or a human growth hormone signal sequence) or a sequence used for purification of a GDNF ligand family protein (e.g., a histidine tag).

GDNF ligand family proteins can be isolated using methods known in the art. Naturally occurring GDNF ligand family proteins can be isolated from cells or tissue sources using standard protein purification techniques. Alternatively, mutated GDNF ligand family proteins can be synthesized chemically using standard peptide synthesis techniques. The synthesis of short amino acid sequences is well established in the peptide art. *See, e.g.,* Stewart, et al., Solid Phase Peptide Synthesis (2d ed., 1984).

In some embodiments, GDNF ligand family proteins are produced by recombinant DNA techniques. For example, a nucleic acid molecule encoding a GDNF ligand family protein can be inserted into a vector, e.g., an expression vector, and the nucleic acid can be introduced into a cell. Suitable cells include, e.g., mammalian cells (such as human cells or CHO cells), fungal cells, yeast cells, insect cells, and bacterial cells (e.g., *E. coli*). When expressed in a recombinant cell, the cell is preferably cultured under conditions allowing for expression of a GDNF ligand family protein. The GDNF ligand family protein can be recovered from a cell suspension if desired. As used herein, "recovered" means that the mutated polypeptide is removed from those components of a cell or culture medium in which it is present prior to the recovery process. The recovery process may include one or more refolding or purification steps. Buffers and methods for inducing folding of a denatured GDNF ligand family protein are described in, e.g., PCT Application Number PCT/US2005/029638.

Variant GDNF ligand family proteins can be constructed using any of several methods known in the art. One such method is site-directed mutagenesis, in which a specific nucleotide (or, if desired a small number of specific nucleotides) is changed in order to change a single amino acid (or, if desired, a small number of predetermined amino acid residues) in the encoded variant GDNF ligand family protein. Many site-directed mutagenesis kits are commercially available. One such kit is the "Transformer Site Directed Mutagenesis Kit" sold by Clontech Laboratories (Palo Alto, CA).

### Pharmaceutical Compositions

A GDNF ligand family protein (e.g., a Neublastin polypeptide, a GDNF polypeptide, a Neurturin polypeptide, or a Persephin polypeptide described herein) can be incorporated into a pharmaceutical composition containing a therapeutically effective amount of the GDNF ligand family protein and an amount of heparin or heparan sulphate that increases serum exposure of the administered GDNF ligand family protein in a treated subject.

Heparin and heparan sulphate are chemically related alpha beta-linked glycosaminoglycans composed of alternating sequences of glucosamine and uronic acid. The size of an individual chain can reach 100 kDa, but normally they are below 50 kDa.

The heparin or heparan sulphate used in the pharmaceutical composition can be unconjugated or conjugated to another molecular entity (e.g., the heparin can be present in the form of a proteoglycan, in which the heparin is conjugated to a protein). Such conjugation is acceptable, so long as it does not eliminate the ability of the heparin or heparan sulphate to increase serum exposure of the administered GDNF ligand family protein in a treated subject. In some embodiments, the heparin or heparan sulphate is covalently linked to the GDNF ligand family protein.

A GDNF ligand family protein and heparin or heparan sulphate can be co-administered simultaneously in a single pharmaceutical composition or can be administered separately via simultaneous or sequential administrations. If administered sequentially, either the heparin or heparan sulphate or the GDNF ligand family protein can be administered first.

In addition to a GDNF ligand family protein and heparin or heparan sulphate, a pharmaceutical composition can also contain one or more adjuvants, excipients, carriers, and/or diluents. Acceptable diluents, carriers and excipients typically do not adversely affect a recipient's homeostasis (e.g., electrolyte balance). Acceptable carriers include biocompatible, inert or bioabsorbable salts, buffering agents, oligo- or polysaccharides, polymers, viscosity-improving agents, preservatives and the like. One exemplary carrier is physiologic saline (0.15 M NaCl, pH 7.0 to 7.4). Another exemplary carrier is 50 mM sodium phosphate, 100 mM sodium chloride. Further details on techniques for formulation and administration of pharmaceutical compositions can be found in, e.g., Remington's Pharmaceutical Sciences (Maack Publishing Co., Easton, Pa.).

A pharmaceutical composition containing a GDNF ligand family protein and heparin or heparan sulphate can be administered by systemic delivery. Pharmaceutical compositions can be formulated such that they are suitable for parenteral and/or non-parenteral administration. Specific administration modalities include subcutaneous, intravenous, intramuscular, intraperitoneal, transdermal, oral, rectal, buccal, nasal, intra-articular, intra-arterial, sub-arachnoid, bronchial, lymphatic, vaginal, and intra-uterine administration.

Administration may be by periodic injections of a bolus of the pharmaceutical composition or may be made more continuous by intravenous or intraperitoneal administration from a reservoir which is external (e.g., an IV bag) or internal (e.g., a bioerodable implant). See, e.g., U.S. Pat. Nos. 4,407,957, 5,798,113, and 5,800,828.

Examples of parenteral delivery systems include ethylene-vinyl acetate copolymer particles, osmotic pumps, implantable infusion systems, pump delivery, encapsulated cell delivery, liposomal delivery, needle-delivered injection, needle-less injection, nebulizer, aeorosolizer, electroporation, and transdermal patch.

Formulations suitable for parenteral administration conveniently contain a sterile aqueous preparation of the GDNF ligand family protein and heparin or heparan sulphate, which preferably is isotonic with the blood of the recipient (*e.g.,* physiological saline solution). Formulations may be presented in unit-dose or multi-dose form.

An exemplary formulation contains a GDNF ligand family protein described herein, heparin or heparan sulphate, and the following buffer components: sodium succinate (e.g., 10 mM); NaCl (e.g., 75 mM); and L-arginine (e.g., 100 mM).

Formulations suitable for oral administration may be presented as discrete units such as capsules, cachets, tablets, or lozenges, each containing a predetermined amount of the GDNF ligand family protein and heparin or heparan sulphate; or a suspension in an aqueous liquor or a non-aqueous liquid, such as a syrup, an elixir, an emulsion, or a draught.

Therapeutically effective amounts of a pharmaceutical composition may be administered to a subject in need thereof in a dosage regimen ascertainable by one of skill in the art. For example, a composition can be administered to the subject, e.g., systemically at a dosage from 0.01µg/kg to 1000 µg/kg body weight of the subject, per dose. In another example, the dosage is from 1 µg/kg to 100 µg/kg body weight of the subject, per dose. In another example, the dosage is from 1 µg/kg to 30 µg/kg body weight of the subject, per dose, e.g., from 3 µg/kg to 10 µg/kg body weight of the subject, per dose.

In order to optimize therapeutic efficacy, a GDNF ligand family protein is first administered at different dosing regimens. The unit dose and regimen depend on factors that include, *e.g.,* the species of mammal, its immune status, the body weight of the mammal. Typically, protein levels in tissue are monitored using appropriate screening assays as part of a clinical testing procedure, e.g., to determine the efficacy of a given treatment regimen.

The frequency of dosing for a GDNF ligand family protein is within the skills and clinical judgement of physicians. Typically, the administration regime is established by clinical trials which may establish optimal administration parameters. However, the practitioner may vary such administration regimes according to the subject's age, health, weight, sex and medical status. The frequency of dosing may be varied depending on whether the treatment is prophylactic or therapeutic.

### Uses for Treatment

A GDNF ligand family protein (e.g., a Neublastin polypeptide, a GDNF polypeptide, a Neurturin polypeptide, or a Persephin polypeptide described herein) is useful for modulating metabolism, growth, differentiation, or survival of a nerve or neuronal cell. In particular, a GDNF ligand family protein (with heparin or heparan sulphate) can be used to treat or alleviate a disorder or disease of a living animal, e.g., a human, which disorder or disease is responsive to the activity of a neurotrophic agent.

The GDNF ligand family proteins disclosed herein (and pharmaceutical compositions comprising same) can be used in the treatment or prevention of a nervous system disorder in a subject (such as a human), by administering to a subject in need thereof a therapeutically effective amount of a GDNF ligand family protein (with heparin or heparan sulphate) or a composition containing a GDNF ligand family protein and heparin or heparan sulphate.

The nervous system disorder can be a peripheral nervous system disorder, such as a peripheral neuropathy or a neuropathic pain syndrome, or a central nervous system disorder.

A GDNF ligand family protein (administered with heparin or heparan sulphate) is useful for treating a defect in a neuron, including lesioned neurons and traumatized neurons. Peripheral nerves that experience trauma include, but are not limited to, nerves of the medulla or of the spinal cord. GDNF ligand family proteins (administered with heparin or heparan sulphate) are useful in the treatment of neurodegenerative disease, e.g., cerebral ischemic neuronal damage; neuropathy, e.g., peripheral neuropathy, Alzheimer's disease, Huntington's disease, Parkinson's disease, amyotrophic lateral sclerosis (ALS). GDNF ligand family proteins (administered with heparin or heparan sulphate) can be used in the treatment of impaired memory, e.g., memory impairment associated with dementia.

In some embodiments, motor neuron diseases such as amyotrophic lateral sclerosis ("ALS") and spinal muscular atrophy can be treated. In other embodiments, the GDNF ligand family proteins (administered with heparin or heparan sulphate) can be used to enhance nerve recovery following traumatic injury.

In some embodiments, the GDNF ligand family proteins and heparin or heparan sulphate (and pharmaceutical compositions comprising same) are used in the treatment of various disorders in the eye, including photoreceptor loss in the retina in patients afflicted with macular degeneration, retinitis pigmentosa, glaucoma, and similar diseases.

In some embodiments, the GDNF ligand family proteins and heparin or heparan sulphate (and pharmaceutical compositions comprising same) are used for treating neuropathic pain, for treating tactile allodynia, for reducing loss of pain sensitivity associated with neuropathy, for treating viral infections and viral-associated neuropathies, and for treating painful diabetic neuropathy. The methods are discussed in detail in the following subsections.

### 1. Treatment of Neuropathic Pain

The GDNF ligand family proteins disclosed herein (and pharmaceutical compositions comprising same) can be used for treating neuropathic pain in a subject comprising administering to the subject an effective amount of a GDNF ligand family protein (with heparin or heparan sulphate) alone, or by also administering to the subject an effective amount of an analgesia-inducing compound selected from the group consisting of opioids, anti-arrhythmics, topical analgesics, local anesthetics, anticonvulsants, antidepressants, corticosteroids and non-steroidal anti-inflammatory drugs (NSAIDS). In one embodiment, the analgesia-inducing compound is an anticonvulsant. In another embodiment, the analgesia-inducing compound is gabapentin ((1-aminomethyl)cyclohexane acetic acid) or pregabalin (S-(+)-4-amino-3-(2-methylpropyl) butanoic acid).

The GDNF ligand family proteins disclosed herein (and pharmaceutical compositions comprising same) can be used in the treatment of pain associated with peripheral neuropathies. Among the peripheral neuropathies which can be treated are trauma-induced neuropathies, e.g., those caused by physical injury or disease state, physical damage to the brain, physical damage to the spinal cord, stroke associated with brain damage, and neurological disorders related to neurodegeneration.

The GDNF ligand family proteins disclosed herein and heparin or heparan sulphate (and pharmaceutical compositions comprising same) can be used in the treatment of a number of peripheral neuropathies, including: (a) trauma-induced neuropathies, (b) chemotherapy-induced neuropathies, (c) toxin-induced neuropathies (including but not limited to neuropathies induced by alcoholism, vitamin B6 intoxication, hexacarbon intoxication, amiodarone, chloramphenicol, disulfiram, isoniazide, gold, lithium, metronidazole, misonidazole, nitrofurantoin), (d) drug-induced neuropathies, including therapeutic drug-induced neuropathic pain (such as caused by anti-cancer agents, particularly anti-cancer agents selected from the group consisting oftaxol, taxotere, cisplatin, nocodazole, vincristine, vindesine and vinblastine; and such as caused by anti-viral agents, particularly anti-viral agents selected from the group consisting of ddI, DDC, d4T, foscarnet, dapsone, metronidazole, and isoniazid), (e) vitamin-deficiency-induced neuropathies (including but not limited to vitamin B12 deficiency, vitamin B6 deficiency, and vitamin E deficiency), (f) idiopathic neuropathies, (g) diabetic neuropathies, (h) pathogen-induced nerve damage, (i) inflammation-induced nerve damage, (j) neurodegeneration, (k) hereditary neuropathy (including but not limited to Friedreich ataxia, familial amyloid polyneuropathy, Tangier disease, Fabry disease), (l) metabolic disorders (including but not limited to renal insufficiency and hypothyroidism), (m) infectious and viral neuropathies (including but not limited to neuropathic pain associated with leprosy, Lyme disease, neuropathic pain associated with infection by a virus, particularly a virus selected from the group consisting of a herpes virus (e.g. herpes zoster which may lead to post-herpetic neuralgia), a human immunodeficiency virus (HIV), and a papilloma virus), (n) auto-immune neuropathies (including but not limited to Guillain-Barre syndrome, chronic inflammatory demyelinating polyneuropathy, monoclonal gammopathy of undetermined significance and polyneuropathy), (o) trigeminal neuralgia and entrapment syndromes (including but not limited to Carpel tunnel), and (p) other neuropathic pain syndromes including post-traumatic neuralgia, phantom limb pain, multiple sclerosis pain, complex regional pain syndromes (including but not limited to reflex sympathetic dystrophy, causalgia), neoplasia- associated pain, vasculitic/angiopathic neuropathy, and sciatica. Neuropathic pain may be manifested as allodynia, hyperalgesia, spontaneous pain or phantom pain.

### 2. Treatment of Tactile Allodynia

The GDNF ligand family proteins disclosed herein and heparin or heparan sulphate (and pharmaceutical compositions comprising same) can be used in the treatment of tactile allodynia in a subject. The term "tactile allodynia" typically refers to the condition in a subject where pain is evoked by stimulation of the skin (e.g. touch) that is normally innocuous.

In some embodiments, tactile allodynia is treated by administering to the subject a pharmaceutically effective amount of a GDNF ligand family protein and heparin or heparan sulphate. In a related embodiment, tactile allodynia may be treated by administering to a subject an effective amount of a GDNF ligand family protein (with heparin or heparan sulphate) alone, or by administering to the subject an effective amount of a GDNF ligand family protein, heparin or heparan sulphate, and an effective amount of an analgesia-inducing compound selected from the group consisting of opioids, anti-arrhythmics, topical analgesics, local anesthetics, anticonvulsants, antidepressants, corticosteroids and NSAIDS. In one embodiment, the analgesia-inducing compound is an anticonvulsant. In another preferred embodiment, the analgesia-inducing compound is gabapentin ((1-aminomethyl)cyclohexane acetic acid) or pregabalin (S-(+)-4-amino-3-(2-methylpropyl)butanoic acid).

In some embodiments, a GDNF ligand family protein and heparin or heparan sulphate is administered in association with a therapeutic agent, including but not limited to an anti-cancer agent or an anti-viral agent. Anti-cancer agents include, but are not limited to, taxol, taxotere, cisplatin, nocodazole, vincristine, vindesine and vinblastine. Anti-viral agents include, but are not limited to, ddI, DDC, d4T, foscarnet, dapsone, metronidazole, and isoniazid.

### 3. Treatment for Reduction of Loss of Pain Sensitivity

In another embodiment, GDNF ligand family proteins disclosed herein and heparin or heparan sulphate (and pharmaceutical compositions comprising same) can be used for reducing the loss of pain sensitivity in a subject afflicted with a neuropathy. In one embodiment, the neuropathy is diabetic neuropathy. In some embodiments, the loss of pain sensitivity is a loss in thermal pain sensitivity. This methods include both prophylactic and therapeutic treatment.

In prophylactic treatment, a GDNF ligand family protein and heparin or heparan sulphate is administered to a subject at risk of developing loss of pain sensitivity (such a subject would be expected to be a subject with an early stage neuropathy). The treatment with a GDNF ligand family protein and heparin or heparan sulphate under such circumstances would serve to treat at-risk patients preventively.

In therapeutic treatment, a GDNF ligand family protein and heparin or heparan sulphate is administered to a subject who has experienced loss of pain sensitivity as a result of affliction with a neuropathy (such a subject would be expected to be a subject with a late stage neuropathy). The treatment with a GDNF ligand family protein and heparin or heparan sulphate under such circumstances would serve to rescue appropriate pain sensitivity in the subject.

### 4. Treatment of Viral Infections and Viral-Associated Neuropathies

Prophylactic treatment of infectious and viral neuropathies is contemplated. Prophylactic treatment is indicated after determination of viral infection and before onset of neuropathic pain. During treatment, a GDNF ligand family protein and heparin or heparan sulphate is administered to prevent appearance of neuropathic pain including but not limited to neuropathic pain associated with leprosy, Lyme disease, neuropathic pain associated with infection by a virus, particularly a virus selected from the group consisting of a herpes virus (and more particularly by a herpes zoster virus, which may lead to post-herpetic neuralgia), a human immunodeficiency virus (HIV), and a papilloma virus). In an alternative embodiment, a GDNF ligand family protein and heparin or heparan sulphate is administered to reduce the severity of neuropathic pain, should it appear.

Symptoms of acute viral infection often include the appearance of a rash. Other symptoms include, for example, the development of persistent pain in the affected area of the body, which is a common complication of a herpes zoster infection (shingles). Post-herpetic neuralgia can last for a month or more, and may appear several months after any rash-like symptoms have disappeared.

### 5. Treatment of Painful Diabetic Neuropathy

Prophylactic treatment of painful diabetic neuropathy is contemplated. Prophylactic treatment of diabetic neuropathies would commence after determination of the initial diagnosis of diabetes or diabetes-associated symptoms and before onset of neuropathic pain. Prophylactic treatment of painful diabetic neuropathy may also commence upon determining that a subject is at risk for developing diabetes or diabetes-associated symptoms. During treatment, a GDNF ligand family protein and heparin or heparan sulphate is administered to prevent appearance of neuropathic pain. In an alternative embodiment, a GDNF ligand family protein and heparin or heparan sulphate is administered to reduce the severity of neuropathic pain that has already appeared.

The following is an example of the practice of the invention. It is not to be construed as limiting the scope of the invention in any way.

### Example: Co-Administration of Heparin and Neublastin

Pre-cannulated (jugular vein) male Sprague Dawley rats were used. Neublastin was administered intravenously via a 1 cc syringe attached to the jugular catheter at a dose of 1 mg/kg either alone or with 16 kDa heparin in a composition containing (in PBS) 5 mM NaCitrate pH 7.0, 150 mM NaCl, and 0.01% Tween-80. The Neublastin polypeptide used in these experiments consisted of the carboxy terminal 113 amino acids of rat wild type Neublastin.

Blood samples were taken from the rats at various time points post dosing. Pharmacokinetics of the administered Neublastin at the various time points were measured by Ternary Complex ELISA.

**Table 1: Concentration of Neublastin Detected in Serum after Intravenous Administration of Neublastin Alone or Co-Administration of Heparin and Neublastin**

| **Minutes after Intravenous Administration** | **Neublastin Alone (concentration in serum, ng/ml)** | **Neublastin + Heparin (concentration in serum, ng/ml)** |
|---|---|---|
| 5 | 3,760 | 5,930 |
| 15 | 1,464 | 6,369 |
| 30 | 287 | 6,018 |
| 60 | 206 | 1,532 |
| 120 | 174 | not determined |

**Table 2: Pharmacokinetic Parameters of Neublastin after Intravenous Administration**

| **Parameters after Intravenous Administration** | **Neublastin Alone** | **Neublastin + Heparin** |
|---|---|---|
| CL | 0.667 L/(hr.kg) | 0.185 L/(hr.kg) |
| T_{1/2} | 0.940 hr | 1.77 hr |
| MRT | 0.913 hr | 0.58 hr |
| V_{SS} | 0.609 L/kg | 0.107 L/kg |
| AUC | 1,500 ng.hr/ml | 5,417 ng.hr/ml |

As detailed in Tables 1 and 2, co-administration of heparin with Neublastin increased (as compared to administration of Neublastin alone) serum exposure of Neublastin, increased the area under the curve (AUC), decreased clearance of Neublastin, and increased the half life of the administered protein.

## Claims

1. A pharmaceutical composition comprising:
(i) a glial cell line-derived neurotrophic factor (GDNF) ligand family protein selected from the group consisting of a Neublastin polypeptide, a GDNF polypeptide, a Neurturin polypeptide, and a Persephin polypeptide; and
(ii) an amount of heparin or heparan sulphate that increases serum exposure of the administered GDNF ligand family protein in a subject,
for use in treating a nervous system disorder via systemic delivery of the pharmaceutical composition.

2. The pharmaceutical composition for use as in claim 1, wherein the GDNF ligand family protein is a Neublastin polypeptide that is at least 80% identical to amino acids 15-113 of SEQ ID NO:1, and wherein the polypeptide, when dimerized, binds to a complex containing GFRalpha3 and RET.

3. The pharmaceutical composition for use as in claim 2, wherein the Neublastin polypeptide is at least 90% identical to amino acids 15-113 of SEQ ID NO:1.

4. The pharmaceutical composition for use as in claim 2, wherein the Neublastin polypeptide comprises amino acids 15-113 of SEQ ID NO:1.

5. The pharmaceutical composition for use as in claim 2 wherein the Neublastin polypeptide consists of the amino acid sequence of SEQ ID NO:1.

6. The pharmaceutical composition for use as in claim 2, wherein the Neublastin polypeptide consists of the carboxy terminal 104 amino acids of SEQ ID NO:1.

7. The pharmaceutical composition for use as in claim 1, wherein the GDNF ligand family protein is a GDNF polypeptide that is at least 80% identical to SEQ ID NO:2, and wherein the polypeptide, when dimerized, binds to a complex containing GFRalpha1 and RET.

8. The pharmaceutical composition for use as in claim 1, wherein the GDNF ligand family protein is a Neurturin polypeptide that is at least 80% identical to SEQ ID NO:3, and wherein the polypeptide, when dimerized, binds to a complex containing GFRalpha2 and RET.

9. The pharmaceutical composition for use as in claim 1, wherein the GDNF ligand family protein is a Persephin polypeptide that is at least 80% identical to SEQ ID NO:4, and wherein the polypeptide, when dimerized, binds to a complex containing GFRalpha4 and RET.

10. The pharmaceutical composition for use as in any one of claims 1 to 9, wherein the nervous system disorder is neuropathic pain.

11. The pharmaceutical composition for use as in any one of claims 1 to 9, wherein the nervous system disorder is a peripheral neuropathy.

12. The pharmaceutical composition for use as in any one of claims 1 to 9, wherein the nervous system disorder is loss of pain sensitivity associated with a neuropathy.

13. The pharmaceutical composition for use as in any one of claims 1 to 12, wherein the GDNF ligand family protein is not conjugated to a polymer.

14. The pharmaceutical composition for use as in any one of claims 1 to 13, wherein the systemic delivery is intravenous administration.

15. The pharmaceutical composition for use as in any one of claims 1 to 13, wherein the systemic delivery is subcutaneous administration.

16. The pharmaceutical composition for use as in any one of claims 1 to 15, wherein the subject is a human.

## Patentansprüche

1. Pharmazeutische Zusammensetzung, umfassend:
(i) ein Protein aus der Familie der von einer Gliazelllinie stammenden neurotrophen Faktor (GDNF) Liganden, ausgewählt aus der Gruppe, die aus einem Neublastinpolypeptid, einem GDNF-Polypeptid, einem Neurturin-Polypeptid und einem Persephin-Polypeptid besteht; und
(ii) eine Menge von Heparin oder Heparansulfat, welche die Serumexposition des verabreichten Proteins aus der Familie der GDNF-Liganden in einem Individuum erhöht,
für die Verwendung beim Behandeln einer Störung des Nervensystems durch systemische Einbringung der pharmazeutischen Zusammensetzung.

2. Pharmazeutische Zusammensetzung für die Verwendung nach Anspruch 1, wobei es sich bei dem Protein aus der Familie der GDNF-Liganden um ein Neublastin-Polypeptid handelt, das zu mindestens 80 % mit den Aminosäuren 15-113 von SEQ ID NO. 1 identisch ist, und wobei das Polypeptid, wenn es dimerisiert ist, an einen Komplex bindet, der GFRalpha3 und RET enthält.

3. Pharmazeutische Zusammensetzung für die Verwendung nach Anspruch 2, wobei das Neublastin-Polypeptid zu mindestens 90 % mit den Aminosäuren 15-113 von SEQ ID NO. 1 identisch ist.

4. Pharmazeutische Zusammensetzung für die Verwendung nach Anspruch 2, wobei das Neublastin-Polypeptid die Aminosäuren 15-113 von SEQ ID NO. 1 umfasst.

5. Pharmazeutische Zusammensetzung für die Verwendung nach Anspruch 2, wobei das Neublastin-Polypeptid aus der Aminosäuresequenz von SEQ ID NO. 1 besteht.

6. Pharmazeutische Zusammensetzung für die Verwendung nach Anspruch 2, wobei das Neublastin-Polypeptid aus den carboxyterminalen 104 Aminosäuren von SEQ ID NO. 1 besteht.

7. Pharmazeutische Zusammensetzung für die Verwendung nach Anspruch 1, wobei es sich bei dem Protein aus der Familie der GDNF-Liganden um ein GDNF-Polypeptid handelt, das zu mindestens 80 % mit SEQ ID NO. 2 identisch ist, und wobei das Polypeptid, wenn es dimerisiert ist, an einen Komplex bindet, der GFRalpha1 und RET enthält.

8. Pharmazeutische Zusammensetzung für die Verwendung nach Anspruch 1, wobei es sich bei dem Protein aus der Familie der GDNF-Liganden um ein Neurturin-Polypeptid handelt, das zu mindestens 80 % mit SEQ ID NO. 3 identisch ist, und wobei das Polypeptid, wenn es dimerisiert ist, an einen Komplex bindet, der GFRalpha2 und RET enthält.

9. Pharmazeutische Zusammensetzung für die Verwendung nach Anspruch 1, wobei es sich bei dem Protein aus der Familie der GDNF-Liganden um ein Persephin-Polypeptid handelt, das zu mindestens 80 % mit SEQ ID NO. 4 identisch ist, und wobei das Polypeptid, wenn es dimerisiert ist, an einen Komplex bindet, der GFRalpha4 und RET enthält.

10. Pharmazeutische Zusammensetzung für die Verwendung nach einem der Ansprüche 1 bis 9, wobei es sich bei der Störung des Nervensystems um neuropathischen Schmerz handelt.

11. Pharmazeutische Zusammensetzung für die Verwendung nach einem der Ansprüche 1 bis 9, wobei es sich bei der Störung des Nervensystems um periphere Neuropathie handelt.

12. Pharmazeutische Zusammensetzung für die Verwendung nach einem der Ansprüche 1 bis 9, wobei es sich bei der Störung des Nervensystems um den Verlust von Schmerzempfindlichkeit in Verbindung mit einer Neuropathie handelt.

13. Pharmazeutische Zusammensetzung für die Verwendung nach einem der Ansprüche 1 bis 12, wobei das Protein aus der Familie der GDNF-Liganden nicht mit einem Polymer konjugiert ist.

14. Pharmazeutische Zusammensetzung für die Verwendung nach einem der Ansprüche 1 bis 13, wobei es sich bei der systemischen Einbringung um intravenöse Verabreichung handelt.

15. Pharmazeutische Zusammensetzung für die Verwendung nach einem der Ansprüche 1 bis 13, wobei es sich bei der systemischen Einbringung um subkutane Verabreichung handelt.

16. Pharmazeutische Zusammensetzung für die Verwendung nach einem der Ansprüche 1 bis 15, wobei es sich bei dem Individuum um einen Menschen handelt.

## Revendications

1. Composition pharmaceutique comprenant:
(i) une protéine de la famille des ligands du facteur neurotrophique (GDNF) dérivé d'une lignée de cellules gliales choisie dans le groupe constitué d'un polypeptide de neublastine, d'un polypeptide du GDNF, d'un polypeptide de neurturine, et d'un polypeptide de perséphine; et
(ii) une quantité d'héparine ou de sulfate d'héparane qui entraîne une augmentation de l'exposition au sérum de la protéine de la famille des ligands du GDNF chez un sujet,
destinée à être utilisée dans le traitement d'un trouble du système nerveux via une administration systémique de la composition pharmaceutique.

2. Composition pharmaceutique destinée à être utilisée selon la revendication 1, dans laquelle la protéine de la famille des ligands du GDNF est un polypeptide de neublastine qui présente une identité d'au moins 80 % avec les acides aminés 15 à 113 de SEQ ID NO : 1, et dans laquelle le polypeptide, lorsqu'il est dimérisé, se lie à un complexe contenant GFRalpha3 et RET.

3. Composition pharmaceutique destinée à être utilisée selon la revendication 2, dans laquelle le polypeptide de neublastine présente une identité d'au moins 90 % avec les acides aminés 15 à 113 de SEQ ID NO : 1.

4. Composition pharmaceutique destinée à être utilisée selon la revendication 2, dans laquelle le polypeptide de neublastine comprend les acides aminés 15 à 113 de SEQ ID NO : 1.

5. Composition pharmaceutique destinée à être utilisée selon la revendication 2, dans laquelle le polypeptide de neublastine est constitué de la séquence d'acides aminés de SEQ ID NO : 1.

6. Composition pharmaceutique destinée à être utilisée selon la revendication 2, dans laquelle le polypeptide de neublastine est constitué des 104 acides aminés carboxy-terminaux de SEQ ID NO : 1.

7. Composition pharmaceutique destinée à être utilisée selon la revendication 1, dans laquelle la protéine de la famille des ligands du GDNF est un polypeptide du GDNF qui présente une identité d'au moins 80 % avec SEQ ID NO : 2, et dans laquelle le polypeptide, lorsqu'il est dimérisé, se lie à un complexe contenant GFRalpha1 et RET.

8. Composition pharmaceutique destinée à être utilisée selon la revendication 1, dans laquelle la protéine de la famille des ligands du GDNF est un polypeptide de neurturine qui présente une identité d'au moins 80 % avec SEQ ID NO : 3, et dans laquelle le polypeptide, lorsqu'il est dimérisé, se lie à un complexe contenant GFRalpha2 et RET.

9. Composition pharmaceutique destinée à être utilisée selon la revendication 1, dans laquelle la protéine de la famille des ligands du GDNF est un polypeptide de perséphine qui présente une identité d'au moins 80 % avec SEQ ID NO : 4, et dans laquelle le polypeptide, lorsqu'il est dimérisé, se lie à un complexe contenant GFRalpha4 et RET.

10. Composition pharmaceutique destinée à être utilisée selon l'une quelconque des revendications 1 à 9, dans laquelle le trouble du système nerveux est une douleur neuropathique.

11. Composition pharmaceutique destinée à être utilisée selon l'une quelconque des revendications 1 à 9, dans laquelle le trouble du système nerveux est une neuropathie périphérique.

12. Composition pharmaceutique destinée à être utilisée selon l'une quelconque des revendications 1 à 9, dans laquelle le trouble du système nerveux est une perte de la sensibilité à la douleur associée à une neuropathie.

13. Composition pharmaceutique destinée à être utilisée selon l'une quelconque des revendications 1 à 12, dans laquelle la protéine de la famille des ligands du GDNF n'est pas conjuguée à un polymère.

14. Composition pharmaceutique destinée à être utilisée selon l'une quelconque des revendications 1 à 13, dans laquelle l'administration systémique est une administration intraveineuse.

15. Composition pharmaceutique destinée à être utilisée selon l'une quelconque des revendications 1 à 13, dans laquelle l'administration systémique est une administration sous-cutanée.

16. Composition pharmaceutique destinée à être utilisée selon l'une quelconque des revendications 1 à 15, dans laquelle le sujet est un humain.


### SEQUENCE LISTING

<110> Biogen Idec MA Inc.
<120> COMPOSITIONS AND METHODS FOR ADMINISTERING GDNF LIGAND FAMILY PROTEINS
<130> 13751-082wO1
<140> PCT/US2007/005366
   <141> 2007-02-27
<150> US 60/778,509
   <151> 2006-03-01
<160> 16
<170> FastSEQ for windows Version 4.0
<210> 1
   <211> 113
   <212> PRT
   <213> Homo sapiens
<400> 1
   Ala Gly Gly Pro Gly Ser Arg Ala Arg Ala Ala Gly Ala Arg Gly Cys
    1               5                  10                  15
   Arg Leu Arg Ser Gln Leu Val Pro Val Arg Ala Leu Gly Leu Gly His
               20                  25                  30
   Arg Ser Asp Glu Leu Val Arg Phe Arg Phe Cys Ser Gly Ser Cys Arg
           35                  40                  45
   Arg Ala Arg Ser Pro His Asp Leu Ser Leu Ala Ser Leu Leu Gly Ala
       50                  55                  60
   Gly Ala Leu Arg Pro Pro Pro Gly Ser Arg Pro Val Ser Gln Pro Cys
   65                  70                  75                  80
   Cys Arg Pro Thr Arg Tyr Glu Ala Val Ser Phe Met Asp Val Asn Ser
                   85                  90                  95
   Thr Trp Arg Thr Val Asp Arg Leu Ser Ala Thr Ala Cys Gly Cys Leu
               100                 105                 110
   Gly
<210> 2
   <211> 134
   <212> PRT
   <213> Homo sapiens
<400> 2
   Ser Pro Asp Lys Gln Met Ala Val Leu Pro Arg Arg Glu Arg Asn Arg
   1               5                   10                  15
   Gln Ala Ala Ala Ala Asn Pro Glu Asn Ser Arg Gly Lys Gly Arg Arg
               20                  25                  30
   Gly Gln Arg Gly Lys Asn Arg Gly Cys Val Leu Thr Ala Ile His Leu
           35                  40                  45
   Asn Val Thr Asp Leu Gly Leu Gly Tyr Glu Thr Lys Glu Glu Leu Ile
       50                  55                  60
   Phe Arg Tyr Cys Ser Gly Ser Cys Asp Ala Ala Glu Thr Thr Tyr Asp
   65                  70                  75                  80
   Lys Ile Leu Lys Asn Leu Ser Arg Asn Arg Arg Leu Val Ser Asp Lys
                   85                  90                  95
   Val Gly Gln Ala Cys Cys Arg Pro Ile Ala Phe Asp Asp Asp Leu Ser
               100                 105                 110
   Phe Leu Asp Asp Asn Leu Val Tyr His Ile Leu Arg Lys His Ser Ala
           115                 120                 125
   Lys Arg Cys Gly Cys Ile
       130
<210> 3
   <211> 102
   <212> PRT
   <213> homo sapiens
<400> 3
   Ala Arg Leu Gly Ala Arg Pro Cys Gly Leu Arg Glu Leu Glu Val Arg
   1               5                   10                  15
   Val Ser Glu Leu Gly Leu Gly Tyr Ala Ser Asp Glu Thr Val Leu Phe
               20                  25                  30
   Arg Tyr Cys Ala Gly Ala Cys Glu Ala Ala Ala Arg Val Tyr Asp Leu
           35                  40                  45
   Gly Leu Arg Arg Leu Arg Gln Arg Arg Arg Leu Arg Arg Glu Arg Val
       50                  55                  60
   Arg Ala Gln Pro Cys Cys Arg Pro Thr Ala Tyr Glu Asp Glu Val Ser
   65                  70                  75                  80
   Phe Leu Asp Ala His Ser Arg Tyr His Thr Val His Glu Leu Ser Ala
                   85                  90                  95
   Arg Glu Cys Ala Cys Val
               100
<210> 4
   <211> 96
   <212> PRT
   <213> Homo sapiens
<400> 4
   Ala Leu Ser Gly Pro Cys Gln Leu Trp Ser Leu Thr Leu Ser Val Ala
   1               5                   10                  15
   Glu Leu Gly Leu Gly Tyr Ala Ser Glu Glu Lys Val Ile Phe Arg Tyr
               20                  25                  30
   Cys Ala Gly Ser Cys Pro Arg Gly Ala Arg Thr Gln His Gly Leu Ala
           35                  40                  45
   Leu Ala Arg Leu Gln Gly Gln Gly Arg Ala His Gly Gly Pro Cys Cys
       50                  55                  60
   Arg Pro Thr Arg Tyr Thr Asp Val Ala Phe Leu Asp Asp Arg His Arg
   65                  70                  75                  80
   Trp Gln Arg Leu Pro Gln Leu Ser Ala Ala Ala Cys Gly Cys Gly Gly
                   85                  90                  95
<210> 5
   <211> 220
   <212> PRT
   <213> Homo sapiens
<400> 5
   Met Glu Leu Gly Leu Gly Gly Leu Ser Thr Leu Ser His Cys Pro Trp
   1               5                   10                  15
   Pro Arg Arg Gln Pro Ala Leu Trp Pro Thr Leu Ala Ala Leu Ala Leu
               20                  25                  30
   Leu Ser Ser Val Ala Glu Ala Ser Leu Gly Ser Ala Pro Arg Ser Pro
           35                  40                  45
   Ala Pro Arg Glu Gly Pro Pro Pro Val Leu Ala Ser Pro Ala Gly His
       50                  55                  60
   Leu Pro Gly Gly Arg Thr Ala Arg Trp Cys Ser Gly Arg Ala Arg Arg
   65                  70                  75                  80
   Pro Pro Pro Gln Pro Ser Arg Pro Ala Pro Pro Pro Pro Ala Pro Pro
                   85                  90                  95
   Ser Ala Leu Pro Arg Gly Gly Arg Ala Ala Arg Ala Gly Gly Pro Gly
               100                 105                 110
   Ser Arg Ala Arg Ala Ala Gly Ala Arg Gly Cys Arg Leu Arg Ser Gln
           115                 120                 125
   Leu Val Pro Val Arg Ala Leu Gly Leu Gly His Arg Ser Asp Glu Leu
       130                 135                 140
   Val Arg Phe Arg Phe Cys Ser Gly Ser Cys Arg Arg Ala Arg Ser Pro
   145                 150                 155                 160
   His Asp Leu Ser Leu Ala Ser Leu Leu Gly Ala Gly Ala Leu Arg Pro
                   165                 170                 175
   Pro Pro Gly Ser Arg Pro Val Ser Gln Pro Cys Cys Arg Pro Thr Arg
               180                 185                 190
   Tyr Glu Ala Val Ser Phe Met Asp Val Asn Ser Thr Trp Arg Thr Val
           195                 200                 205
   Asp Arg Leu Ser Ala Thr Ala Cys Gly Cys Leu Gly
       210                 215                 220
<210> 6
   <211> 224
   <212> PRT
   <213> mus musculus
<400> 6
   Met Glu Leu Gly Leu Ala Glu Pro Thr Ala Leu Ser His Cys Leu Arg
    1               5                  10                  15
   Pro Arg Trp Gln Ser Ala Trp Trp Pro Thr Leu Ala Val Leu Ala Leu
               20                  25                  30
   Leu Ser Cys Val Thr Glu Ala Ser Leu Asp Pro Met Ser Arg Ser Pro
           35                  40                  45
   Ala Ala Arg Asp Gly Pro Ser Pro Val Leu Ala Pro Pro Thr Asp His
       50                  55                  60
   Leu Pro Gly Gly His Thr Ala His Leu Cys Ser Glu Arg Thr Leu Arg
   65                  70                  75                      80
   Pro Pro Pro Gln Ser Pro Gln Pro Ala Pro Pro Pro Pro Gly Pro Ala
                   85                  90                  95
   Leu Gln Ser Pro Pro Ala Ala Leu Arg Gly Ala Arg Ala Ala Arg Ala
               100                 105                 110
   Gly Thr Arg Ser Ser Arg Ala Arg Thr Thr Asp Ala Arg Gly Cys Arg
           115                 120                 125
   Leu Arg Ser Gln Leu Val Pro Val Ser Ala Leu Gly Leu Gly His Ser
       130                 135                 140
   Ser Asp Glu Leu Ile Arg Phe Arg Phe Cys Ser Gly Ser Cys Arg Arg
   145                 150                 155                 160
   Ala Arg Ser Gln His Asp Leu Ser Leu Ala Ser Leu Leu Gly Ala Gly
                   165                 170                 175
   Ala Leu Arg Ser Pro Pro Gly Ser Arg Pro Ile Ser Gln Pro Cys Cys
               180                 185                 190
   Arg Pro Thr Arg Tyr Glu Ala Val Ser Phe Met Asp Val Asn Ser Thr
           195                 200                 205
   Trp Arg Thr Val Asp His Leu Ser Ala Thr Ala Cys Gly Cys Leu Gly
       210                 215                 220
<210> 7
   <211> 224
   <212> PRT
   <213> Rattus norvegicus
<400> 7
   Met Glu Leu Gly Leu Gly Glu Pro Thr Ala Leu Ser His Cys Leu Arg
    1               5                  10                  15
   Pro Arg Trp Gln Pro Ala Leu Trp Pro Thr Leu Ala Ala Leu Ala Leu
               20                  25                  30
   Leu Ser Ser Val Thr Glu Ala Ser Leu Asp Pro Met Ser Arg Ser Pro
           35                  40                  45
   Ala Ser Arg Asp Val Pro Ser Pro Val Leu Ala Pro Pro Thr Asp Tyr
       50                  55                  60
   Leu Pro Gly Gly His Thr Ala His Leu Cys Ser Glu Arg Thr Leu Arg
   65                  70                  75                      80
   Pro Pro Pro Gln Ser Pro Gln Pro Ala Pro Pro Pro Pro Gly Pro Ala
                   85                  90                  95
   Leu Gln Ser Pro Pro Ala Ala Leu Arg Gly Ala Arg Ala Ala Arg Ala
               100                 105                 110
   Gly Thr Arg Ser Ser Arg Ala Arg Ala Thr Asp Ala Arg Gly Cys Arg
           115                 120                 125
   Leu Arg Ser Gln Leu Val Pro Val Ser Ala Leu Gly Leu Gly His Ser
       130                 135                 140
   Ser Asp Glu Leu Ile Arg Phe Arg Phe Cys Ser Gly Ser Cys Arg Arg
   145                 150                 155                 160
   Ala Arg Ser Pro His Asp Leu Ser Leu Ala Ser Leu Leu Gly Ala Gly
                   165                 170                 175
   Ala Leu Arg Ser Pro Pro Gly Ser Arg Pro Ile Ser Gln Pro Cys Cys
               180                 185                 190
   Arg Pro Thr Arg Tyr Glu Ala Val Ser Phe Met Asp Val Asn Ser Thr
           195                 200                 205
   Trp Arg Thr Val Asp His Leu Ser Ala Thr Ala Cys Gly Cys Leu Gly
       210                 215                 220
<210> 8
   <211> 211
   <212> PRT
   <213> Homo sapiens
<400> 8
   Met Lys Leu Trp Asp Val Val Ala Val Cys Leu Val Leu Leu His Thr
   1               5                   10                  15
   Ala Ser Ala Phe Pro Leu Pro Ala Gly Lys Arg Pro Pro Glu Ala Pro
               20                  25                  30
   Ala Glu Asp Arg Ser Leu Gly Arg Arg Arg Ala Pro Phe Ala Leu Ser
           35                  40                  45
   Ser Asp Ser Asn Met Pro Glu Asp Tyr Pro Asp Gln Phe Asp Asp Val
       50                  55                  60
   Met Asp Phe Ile Gln Ala Thr Ile Lys Arg Leu Lys Arg Ser Pro Asp
   65                  70                  75                  80
   Lys Gln Met Ala Val Leu Pro Arg Arg Glu Arg Asn Arg Gln Ala Ala
                   85                  90                  95
   Ala Ala Asn Pro Glu Asn Ser Arg Gly Lys Gly Arg Arg Gly Gln Arg
               100                 105                 110
   Gly Lys Asn Arg Gly Cys Val Leu Thr Ala Ile His Leu Asn Val Thr
           115                 120                 125
   Asp Leu Gly Leu Gly Tyr Glu Thr Lys Glu Glu Leu Ile Phe Arg Tyr
       130                 135                 140
   Cys Ser Gly Ser Cys Asp Ala Ala Glu Thr Thr Tyr Asp Lys Ile Leu
   145                 150                 155                 160
   Lys Asn Leu Ser Arg Asn Arg Arg Leu Val Ser Asp Lys Val Gly Gln
                   165                 170                 175
   Ala Cys Cys Arg Pro Ile Ala Phe Asp Asp Asp Leu Ser Phe Leu Asp
               180                 185                 190
   Asp Asn Leu Val Tyr His Ile Leu Arg Lys His Ser Ala Lys Arg Cys
           195                 200                 205
   Gly Cys Ile
       210
<210> 9
   <211> 240
   <212> PRT
   <213> Mus musculus
<400> 9
   Met Gly Phe Gly Pro Leu Gly Val Asn Val Gln Leu Gly Val Tyr Gly
   1               5                   10                  15
   Asp Arg Ile Arg Gly Ala Ala Ala Gly Arg Asp Ser Lys Met Lys Leu
               20                  25                  30
   Trp Asp Val Val Ala Val Cys Leu Val Leu Leu His Thr Ala Ser Ala
           35                  40                  45
   Phe Pro Leu Pro Ala Gly Lys Arg Leu Leu Glu Ala Pro Ala Glu Asp
       50                  55                  60
   His Ser Leu Gly His Arg Arg Val Pro Phe Ala Leu Thr Ser Asp Ser
   65 70 75 80
   Asn Met Pro Glu Asp Tyr Pro Asp Gln Phe Asp Asp Val Met Asp Phe
   85 90 95
   Ile Gln Ala Thr Ile Lys Arg Leu Lys Arg Ser Pro Asp Lys Gln Ala
   100 105 110
   Ala Ala Leu Pro Arg Arg Glu Arg Asn Arg Gln Ala Ala Ala Ala Ser
   115 120 125
   Pro Glu Asn Ser Arg Gly Lys Gly Arg Arg Gly Gln Arg Gly Lys Asn
   130 135 140
   Arg Gly Cys Val Leu Thr Ala Ile His Leu Asn Val Thr Asp Leu Gly
   145 150 155 160
   Leu Gly Tyr Glu Thr Lys Glu Glu Leu Ile Phe Arg Tyr Cys Ser Gly
   165 170 175
   Ser Cys Glu Ser Ala Glu Thr Met Tyr Asp Lys Ile Leu Lys Asn Leu
   180 185 190
   Ser Arg Ser Arg Arg Leu Thr Ser Asp Lys Val Gly Gln Ala Cys Cys
   195 200 205
   Arg Pro Val Ala Phe Asp Asp Asp Leu Ser Phe Leu Asp Asp Asn Leu
   210 215 220
   Val Tyr His Ile Leu Arg Lys His Ser Ala Lys Arg Cys Gly Cys Ile
   225 230 235 240
<210> 10
   <211> 211
   <212> PRT
   <213> Rattus norvegicus
<400> 10
   Met Lys Leu Trp Asp Val Val Ala Val Cys Leu Val Leu Leu His Thr
   1 5 10 15
   Ala Ser Ala Phe Pro Leu Pro Ala Gly Lys Arg Leu Leu Glu Ala Pro
   20 25 30
   Ala Glu Asp His Ser Leu Gly His Arg Arg Val Pro Phe Ala Leu Thr
   35 40 45
   Ser Asp Ser Asn Met Pro Glu Asp Tyr Pro Asp Gln Phe Asp Asp Val
   50 55 60
   Met Asp Phe Ile Gln Ala Thr Ile Lys Arg Leu Lys Arg Ser Pro Asp
   65 70 75 80
   Lys Gln Ala Ala Ala Leu Pro Arg Arg Glu Arg Asn Arg Gln Ala Ala
   85 90 95
   Ala Ala Ser Pro Glu Asn Ser Arg Gly Lys Gly Arg Arg Gly Gln Arg
   100 105 110
   Gly Lys Asn Arg Gly Cys Val Leu Thr Ala Ile His Leu Asn Val Thr
   115 120 125
   Asp Leu Gly Leu Gly Tyr Glu Thr Lys Glu Glu Leu Ile Phe Arg Tyr
   130 135 140
   Cys Ser Gly Ser Cys Glu Ala Ala Glu Thr Met Tyr Asp Lys Ile Leu
   145 150 155 160
   Lys Asn Leu Ser Arg Ser Arg Arg Leu Thr Ser Asp Lys Val Gly Gln
   165 170 175
   Ala Cys Cys Arg Pro Val Ala Phe Asp Asp Asp Leu Ser Phe Leu Asp
   180 185 190
   Asp Ser Leu Val Tyr His Ile Leu Arg Lys His Ser Ala Lys Arg Cys
   195 200 205
   Gly Cys Ile
   210
<210> 11
   <211> 197
   <212> PRT
   <213> Homo sapiens
<400> 11
   Met Gln Arg Trp Lys Ala Ala Ala Leu Ala Ser Val Leu Cys Ser Ser
    1               5                  10                  15
   Val Leu Ser Ile Trp Met Cys Arg Glu Gly Leu Leu Leu Ser His Arg
               20                  25                  30
   Leu Gly Pro Ala Leu Val Pro Leu His Arg Leu Pro Arg Thr Leu Asp
           35                  40                  45
   Ala Arg Ile Ala Arg Leu Ala Gln Tyr Arg Ala Leu Leu Gln Gly Ala
       50                  55                  60
   Pro Asp Ala Met Glu Leu Arg Glu Leu Thr Pro Trp Ala Gly Arg Pro
   65                  70                  75                  80
   Pro Gly Pro Arg Arg Arg Ala Gly Pro Arg Arg Arg Arg Ala Arg Ala
                   85                  90                  95
   Arg Leu Gly Ala Arg Pro Cys Gly Leu Arg Glu Leu Glu Val Arg Val
               100                 105                 110
   Ser Glu Leu Gly Leu Gly Tyr Ala Ser Asp Glu Thr Val Leu Phe Arg
           115                 120                 125
   Tyr Cys Ala Gly Ala Cys Glu Ala Ala Ala Arg Val Tyr Asp Leu Gly
       130                 135                 140
   Leu Arg Arg Leu Arg Gln Arg Arg Arg Leu Arg Arg Glu Arg Val Arg
   145                 150                 155                 160
   Ala Gln Pro Cys Cys Arg Pro Thr Ala Tyr Glu Asp Glu Val Ser Phe
                   165                 170                 175
   Leu Asp Ala His Ser Arg Tyr His Thr Val His Glu Leu Ser Ala Arg
               180                 185                 190
   Glu Cys Ala Cys Val
           195
<210> 12
   <211> 195
   <212> PRT
   <213> Mus muscukus
<400> 12
   Met Arg Arg Trp Lys Ala Ala Ala Leu Val Ser Leu Ile Cys Ser Ser
    1               5                  10                  15
   Leu Leu Ser Val Trp Met Cys Gln Glu Gly Leu Leu Leu Gly His Arg
               20                  25                  30
   Leu Gly Pro Ala Leu Ala Pro Leu Arg Arg Pro Pro Arg Thr Leu Asp
           35                  40                  45
   Ala Arg Ile Ala Arg Leu Ala Gln Tyr Arg Ala Leu Leu Gln Gly Ala
       50                  55                  60
   Pro Asp Ala Val Glu Leu Arg Glu Leu Ser Pro Trp Ala Ala Arg Ile
   65                  70                  75                  80
   Pro Gly Pro Arg Arg Arg Ala Gly Pro Arg Arg Arg Arg Ala Arg Pro
                   85                  90                  95
   Gly Ala Arg Pro Cys Gly Leu Arg Glu Leu Glu Val Arg Val Ser Glu
               100                 105                 110
   Leu Gly Leu Gly Tyr Thr Ser Asp Glu Thr Val Leu Phe Arg Tyr Cys
           115                 120                 125
   Ala Gly Ala Cys Glu Ala Ala Ile Arg Ile Tyr Asp Leu Gly Leu Arg
       130                 135                 140
   Arg Leu Arg Gln Arg Arg Arg Val Arg Arg Glu Arg Ala Arg Ala His
   145                 150                 155                 160
   Pro Cys Cys Arg Pro Thr Ala Tyr Glu Asp Glu Val Ser Phe Leu Asp
                   165                 170                 175
   Val His Ser Arg Tyr His Thr Leu Gln Glu Leu Ser Ala Arg Glu Cys
               180                 185                 190
   Ala Cys Val
           195
<210> 13
   <211> 195
   <212> PRT
   <213> Rattus norvegicus
<400> 13
   Met Arg Cys Trp Lys Ala Ala Ala Leu Val Ser Leu Ile Cys Ser Ser
   1               5                   10                  15
   Leu Leu Ser Val Trp Met Cys Gln Glu Gly Leu Leu Leu Gly His Arg
               20                  25                  30
   Leu Gly Pro Ala Leu Ala Pro Leu Arg Arg Pro Pro Arg Thr Leu Asp
           35                  40                  45
   Ala Arg Ile Ala Arg Leu Ala Gln Tyr Arg Ala Leu Leu Gln Gly Ala
       50                  55                  60
   Pro Asp Ala Val Glu Leu Arg Glu Leu Ser Pro Trp Val Ala Arg Pro
   65                  70                  75                  80
   Ser Gly Pro Arg Arg Arg Ala Gly Pro Arg Arg Arg Arg Ala Arg Pro
                   85                  90                  95
   Gly Ser Arg Pro Cys Gly Leu Arg Glu Leu Glu Val Arg Val Ser Glu
               100                 105                 110
   Leu Gly Leu Gly Tyr Thr Ser Asp Glu Thr Val Leu Phe Arg Tyr Cys
           115                 120                 125
   Ala Gly Ala Cys Glu Ala Ala Ile Arg Ile Tyr Asp Leu Gly Leu Arg
       130                 135                 140
   Arg Leu Arg Gln Arg Arg Arg Val Arg Lys Glu Arg Val Arg Ala His
   145                 150                 155                 160
   Pro Cys Cys Arg Pro Thr Ala Tyr Glu Asp Glu Val Ser Phe Leu Asp
                   165                 170                 175
   Val His Ser Arg Tyr His Thr Leu Gln Glu Leu Ser Ala Arg Glu Cys
               180                 185                 190
   Ala Cys Val
           195
<210> 14
   <211> 156
   <212> PRT
   <213> Homo sapiens
<400> 14
   Met Ala Val Gly Lys Phe Leu Leu Gly Ser Leu Leu Leu Leu Ser Leu
   1               5                   10                  15
   Gln Leu Gly Gln Gly Trp Gly Pro Asp Ala Arg Gly Val Pro Val Ala
               20                  25                  30
   Asp Gly Glu Phe Ser Ser Glu Gln Val Ala Lys Ala Gly Gly Thr Trp
           35                  40                  45
   Leu Gly Thr His Arg Pro Leu Ala Arg Leu Arg Arg Ala Leu Ser Gly
       50                  55                  60
   Pro Cys Gln Leu Trp Ser Leu Thr Leu Ser Val Ala Glu Leu Gly Leu
   65                  70                  75                  80
   Gly Tyr Ala Ser Glu Glu Lys Val Ile Phe Arg Tyr Cys Ala Gly Ser
                   85                  90                  95
   Cys Pro Arg Gly Ala Arg Thr Gln His Gly Leu Ala Leu Ala Arg Leu
               100                 105                 110
   Gln Gly Gln Gly Arg Ala His Gly Gly Pro Cys Cys Arg Pro Thr Arg
           115                 120                 125
   Tyr Thr Asp Val Ala Phe Leu Asp Asp Arg His Arg Trp Gln Arg Leu
       130                 135                 140
   Pro Gln Leu Ser Ala Ala Ala Cys Gly Cys Gly Gly
   145                 150                 155
<210> 15
   <211> 156
   <212> PRT
   <213> Mus musculus
<400> 15
   Met Ala Ala Gly Arg Leu Arg Ile Leu Cys Leu Leu Leu Leu Ser Leu
   1               5                   10                  15
   His Pro Ser Leu Gly Trp Val Leu Asp Leu Gln Glu Ala Ser Val Ala
               20                  25                  30
   Asp Lys Leu Ser Phe Gly Lys Met Ala Glu Thr Arg Gly Thr Trp Thr
           35                  40                  45
   Pro His Gln Gly Asn Asn His Val Arg Leu Pro Arg Ala Leu Ala Gly
       50                  55                  60
   Ser Cys Arg Leu Trp Ser Leu Thr Leu Pro Val Ala Glu Leu Gly Leu
   65                  70                  75                  80
   Gly Tyr Ala Ser Glu Glu Lys Val Ile Phe Arg Tyr Cys Ala Gly Ser
                   85                  90                  95
   Cys Pro Gln Glu Ala Arg Thr Gln His Ser Leu Val Leu Ala Arg Leu
               100                 105                 110
   Arg Gly Arg Gly Arg Ala His Gly Arg Pro Cys Cys Gln Pro Thr Ser
           115                 120                 125
   Tyr Ala Asp Val Thr Phe Leu Asp Asp Gln His His Trp Gln Gln Leu
       130                 135                 140
   Pro Gln Leu Ser Ala Ala Ala Cys Gly Cys Gly Gly
   145                 150                 155
<210> 16
   <211> 156
   <212> PRT
   <213> Rattus norvegicus
<400> 16
   Met Ala Ala Gly Arg Leu Arg Ile Leu Phe Leu Leu Leu Leu Ser Leu
   1               5                   10                  15
   His Leu Gly Leu Gly Trp Val Leu Asp Leu Gln Glu Ala Pro Ala Ala
               20                  25                  30
   Asp Glu Leu Ser Ser Gly Lys Met Ala Glu Thr Gly Arg Thr Trp Lys
           35                  40                  45
   Pro His Gln Gly Asn Asn Asn Val Arg Leu Pro Arg Ala Leu Pro Gly
       50                  55                  60
   Leu Cys Arg Leu Trp Ser Leu Thr Leu Pro Val Ala Glu Leu Gly Leu
   65                  70                  75                  80
   Gly Tyr Ala Ser Glu Glu Lys Ile Ile Phe Arg Tyr Cys Ala Gly Ser
                   85                  90                  95
   Cys Pro Gln Glu Val Arg Thr Gln His Ser Leu Val Leu Ala Arg Leu
               100                 105                 110
   Arg Gly Gln Gly Arg Ala His Gly Arg Pro Cys Cys Gln Pro Thr Ser
           115                 120                 125
   Tyr Ala Asp Val Thr Phe Leu Asp Asp His His His Trp Gln Gln Leu
       130                 135                 140
   Pro Gln Leu Ser Ala Ala Ala Cys Gly Cys Gly Gly
   145                 150                 155